# EUROPEAN PATENT SPECIFICATION

(11) **EP 1 272 152 B1**
(45) Date of publication and mention of the grant of the patent: **07.03.2012**
(21) Application number: 01925804.5
(22) Date of filing: 03.04.2001
(51) Int. Cl.: A61K 6/00, A61K 8/43, A61Q 11/00

(54) **Use of chlorhexidine in the prevention of root caries**
Verwendung von Chlorhexidine zur Verhinderung von Wurzelkaries
Utilisation de la chlorhexidine pour la prevention de la carie du cement

(30) Priority: 03.04.2000 US 194559 P
(43) Date of publication of application: 08.01.2003
(73) Proprietor: CHX Technologies Inc., Toronto, Ontario M9A 1B1 (CA)
(72) Inventor: SYMINGTON, John, M., Etobicoke, Ontario M9C 3J8 (CA); SYMINGTON, Alison, Toronto, Ontario M4G 2P8 (CA); TEN CATE, Arnold, R., New Market Ontario L3Y8T4 (CA); PERRY, Oliver, R., Toronto, Ontario M6P 3C3 (CA)
(74) Representative: Jacob, Reuben Ellis
(86) International application number: PCT/IB2001/000794
(87) International publication number: WO 2001/074321

(56) References cited:
- EP-A- 0 951 893
- WO-A-89/10736
- WO-A1-95/22308
- US-A- 3 887 701
- US-A- 4 883 534
- US-A- 5 178 870
- DATABASE WPI Week 199435 Derwent Publications Ltd., London, GB; AN 1994-284020 XP002188705 & RU 2 008 842 C (SAMARA STOMATOLOGICAL POLYCLINIC), 15 March 1994 (1994-03-15)
- J. DENT. RES., vol. 73, no. 3, 1 March 1994 (1994-03-01), pages 672-681,
- GERODONTOLOGY, vol. 16, no. 1, 1999, pages 37-46,

## Description

The invention relates to the prevention of the destruction of exposed cementum tissue on human tooth root surfaces and the reduction of inflammation of gingival tissues, and more particularly, to the topical application of the antimicrobial agent chlorhexidine to reduce the increment of root caries and to reduce gingival inflammation caused by root caries or by an infection with lactobacillus sp.

Periodontal disease and dental caries are chronic conditions found in the dentition of a significant proportion of the general adult population. There is, therefore, tremendous incentive to develop preventative treatments for these diseases. Both diseases are now generally accepted as bacterial infections found in the dental plaque that forms on teeth and in periodontal pockets.

Periodontal disease refers generally to inflammatory conditions that attack the gingiva and the underlying alveolar bone supporting the teeth. Anaerobic bacteria, such as *P. gingivalis* and black-pigmented *Bacteroides*, proliferate in the gingival crevice and produce enzymes, toxins, and noxious metabolites, that accumulate. These bacterial byproducts irritate the gingival tissue and initiate a localized inflammation (gingivitis). The inflamed tissue releases enzymes that destroy the collagenous tissue and alveolar bone (periodontitis). If left untreated, this condition will eventually result in loss of the affected tooth.

Dental caries refers to tooth decay or the destruction of tooth surfaces by acids produced by cariogenic bacteria (also known as demineralization). As used in the dental research literature, the term "caries" is used generically to encompass the destruction via demineralization of all tooth surfaces. However, there are two surfaces, or tissues of the tooth, that may be attacked, the enamel on the crown of the tooth and the cementum on the root of the tooth. Moreover, dental research indicates that cariogenic organisms display selectivity as to which tooth surface they will attack and the mix and role of the organisms change as carious lesions progress.

A convenient comparison table outlining the differences between these two tissues is available in Ten Cate, "Oral Histology," Mosby, Toronto (1998) at page 287. Enamel is a highly mineralized tissue produced by epithelial cells and contains less than 4% organic matter and water. Cementum, on the other hand, is a connective tissue containing approximately 50% organic material that contains significantly less mineralized tissue by weight than enamel. Since cementum tissue differs substantially from the enamel tissue in the crown of a human tooth, the associated demineralization processes for these two tissues differ substantially. For example, the drop in pH necessary to initiate decalcification/demineralization (6.7) in cementum is more than an order of magnitude less than the pH (5.4) necessary to initiate demineralization of enamel. As a result, cementum is more soluble than enamel, and therefore, root lesions progress faster than coronal caries. Moreover, demineralization continues for a longer duration, for each acid challenge, than it does in enamel. Prevention of root caries is important since these lesions are more difficult to restore and lead to tooth loss if untreated. Furthermore, progressive exposure of cementum with age makes it more vulnerable to demineralization associated with certain microorganisms in the oral cavity. As a result, preventative strategies that work on enamel may not be appropriate, or optimum, for cementum, and *vice versa.* Similarly, strategies to prevent caries in adolescents may not be optimum for preventing caries in adults or elderly.

While the dental research literature implicates *Streptococcus mutans* in the initiation and progressive demineralization of both enamel and cementum tissues, there is also a significant body of literature that associates Lactobacilli, and most notably *Lactobacillus casei,* with root caries. See, for example, Beck, Adv. Dent. Res., Vol. 7, No.1, pp. 42-51 (1993); Faine, et al., Special Care in Dentistry. Vol. 12, No. 4, pp. 177-182 (1992); Hunt, et al., Special Care in Dentistry, Vol. 12, No. 4, pp. 149ff (1992).

In fact, an article by Loesche, et al., Gerodontology, Vol. 16, No. 1 (1999), presented results of a study that demonstrated that *S. mutans* were significantly associated with coronal surface decay in older individuals (similar in average age to the xerostomia trial described hereinbelow as Clinical Study 1) and that Lactobacilli were significantly associated with root surface decay. The odds/ratio for oral/dental parameters which are significantly associated with decay in elderly individuals, according to the Loesche, *et al.* study, appears below in Table 1.

**TABLE 1**

| Odds ratio for oral/dental parameters which are significantly associated with decay in elderly individuals | | | |
|---|---|---|---|
| factor | Any decay | Enamel surface decay | Cementum decay |
| *S. mutans* | 1.12 | 1.12 | |
| *Lactobacillus sp.* | 1.09 | | 1.24 |

The locus, nature, pathology, etiology, and restorative outcomes of tooth decay, particularly at the root surface, are different in the adult than in the young. In addition to the foregoing, there is a high incidence of caries in patients with salivary hypofunction resulting from chronic medication, systemic disease (*e.g.*, arthritis, lupus, Sjogren's Syndrome) or radiation therapy which conditions are far more prevalent in the adult or older dental patient population. This same population also has a higher incidence of periodontal disease and gingival inflammation.

These is, therefore, a need for a safe and effective preventative treatment for caries, particularly root caries, and gingival inflammation, in "at risk" patient groups, particularly older adults and older adults with salivary hypofunction. This "at risk" population accounts for a minority of dental patients, but a majority of the disease incidence.

Chlorhexidine is a bis biguanide antiseptic and disinfectant that has bactericidal and bacteriostatic action against a wide range of gram-positive and gram-negative bacteria. Chlorhexidine has been used to control the development of caries and is believed to be effective in controlling *S. mutans* infections. U.S. Patent No. 4,496, 322 describes a dental varnish containing an antimicrobial agent, specifically chlorhexidine acetate, a benzoin gum, and an orally-acceptable solvent that, when applied to teeth, dries to a film, that provides sustained release of the antimicrobial agent. An improvement on this technology was described in U.S. Patent No. 4,883,534 which further provided a sealing composition, applied to the varnish, to extend the length of the antimicrobial protection provided by the varnish. The aforementioned patents are directed to the reduction of cariogenic bacteria, specifically *S. mutans*, on tooth surfaces for long periods of time. These patents provide no controlled clinical evidence of the caries or gingival outcomes of this antimicrobial effect, and in particular, these patents provide no indication or reference to the effect on the preservation of cementum tissues from the reduction of bacterial titers in the oral cavity. In fact, based on these patents and the dental research literature, root caries are associated with Lactobacillus, and therefore, one would not expect the application of chlorhexidine dental varnish to have an effect on root caries prevention or control. Nor would one expect the application of chlorhexidine varnish to have an effect on the prevention and control of gingival inflammation resulting from the known periodontal pathogens.

European patent application EP 0 951 893 discloses the use of chlorhexidine as an antibacterial active agent in a filling composition used to fill the dental roots and is directed to a filling composition. However, the inventors' herein are not proposing a filling material but rather a temporary coating on the surface of the tooth.

US Patent US 5,178,870 discloses the use of chlorhexidine as an active agent in a varnish against caries and root surface caries. This document reports clinical results from a small double-blind, controlled study of adult periodontal patients using a concentrated 50% w/v chlorhexidine diacetate varnish which was applied only to the surfaces of denuded tooth roots. The results were measured are based primarily on cavitated lesions and filled lesions, known as Decayed and Filled Surfaces (DFS). However, there is no indication of results based on non-cavitated secondary (or recurrent) lesions which occur around existing filled surfaces, at lower concentrations of chlorhexidine.

It is, therefore, an object of the invention to provide a medicament for the preventative treatment for caries, particularly root caries.

It is also an object of the invention to provide a medicament for the treatment for root caries and gingival inflammation caused by root caries or by an infection with lactobacillus sp. that is particularly useful for older adults, especially those with saliva bypofunction or xerostomia.

The foregoing and other objects, features and advantages are achieved by this invention which provides a medicament for preventing root caries and/or preventing and controlling gingival inflammation caused by root caries or by an infection with lactobacillus sp. particularly in patients with active caries an the cementum or cementum-enamel junction or gingival inflammation (gingivitis) or at risk for these conditions. As used herein, the term "preventing root caries" means preventing the destruction of exposed cementum and associated exposed enamel at the cementum-enamel junction on tooth root surfaces.

The method using said medicament is particularly applicable to the preventive treatment of older patient populations, and patient populations that are "at risk" for root caries and/or gingival inflammation. The method was demonstrated to be particularly successful in preventing root caries and gingival inflammation in an "at risk" patient population including the elderly (average age about 60 years old) and, particularly, the elderly (and others) with salivary hypofunction, or xerostomia.

The method comprises contacting the appropriate tooth surface with an effective amount of the antimicrobial agent, chlorhexidine, preferably in a form that provides sustained release of chlorhexidine into the saliva and salivary reservoirs in the oral cavity, such as a dental varnish. The appropriate "tooth surface" for the practice of the present invention includes from the cementum down to the gingival margin, and specifically including any exposed dentine between the enamel and the cementum at the cemento-enamel or dentino-enamel junctions.

In the practice of the invention, the tooth surfaces are preferably contacted with a non toxic, topical solution of chlorhexidine, and preferably up to and including 10% (w/v) chlorhexidine or chlorhexidine salt in solution according to a predetermined dosing schedule. Since it is preferred that chlorhexidine be administered in a form suitable for sustained release of the antimicrobial agent over time, a binder, such as a varnish base or resin, illustratively benzoin gum, is typically employed to create a film-forming solution in an orally-acceptable solvent In a specific preferred embodiment, the topical solution is a varnish comprising 10% (w/v) chlorhexidine, 20% (w/v) Sumatra benzoin, and 70% (w/v) ethanol. This varnish is commercially available in Canada under the trade mark CHLORZOIN (Stage 1) from CHX Technologies, Inc., Toronto, Canada.

Of course, the topical solution of chlorhexidine may comprise other non-toxic, biologically acceptable solutions of chlorhexidine and/or salts of chlorhexidine that are suitable for topical application and internal use. Presently known and used salts of chlorhexidine include chlorhexidine acetate, chlorhexidine hydrochloride, and chlorhexidine gluconate. Other orally-acceptable solvents include, but are not limited to, methanol, n-propanol, and isopropanol. The topical solution may also include other ingredients, such as flavorants or flavor masking agents. The topical solution of chlorhexidine is formed by making an admixture of all of the ingredients.

Application of the topical solution of chlorhexidine is most preferably followed by application of a sealant over the chlorhexidine-containing film. In a specific preferred embodiment, the sealant is 29% (w/v) medical-grade polyurethane in 49 % (w/v) acetone and 22% (w/v) ethyl acetate. This solution is commercially available in Canada under the trade mark CHLORZOIN (Stage II) from CHX Technologies, Inc., Toronto, Canada. Of course, the sealant can be any solvated non-toxic biodegradable polymer, such as polyurethane or polylactate, that forms a film and is capable of extending retention of the chlorhexidine-containing film at the site of application and/or retarding release of chlorhexidine.

The sealant may further include other therapeutic components, illustratively a fluoride salt. Since fluoride is known to promote remineralization or construction of enamel surfaces whereas chlorhexidine controls demineralization or destruction of the enamel surfaces, the combination of the chlorhexidine-containing varnish and a fluoride-containing sealant may work in combination to cause a significant beneficial effect. The sustained release of fluoride into the saliva and salivary reservoirs in the oral cavity may actually promote healing of carious lesions. Fluoride-containing sealants may be made by dissolving the desired fluoride salt (*e.g*., sodium fluoride, sodium monofluorophosphate, or stannous fluoride) in a solvent or mixture of co-solvents. Fluoride-containing sealants are also commercially available, such as DUROFLUOR by Pharmascience, Montreal, Canada (NaF, 50 mg/ml) or FLUOR-PROTECTOR by Ivoclar-Vivadent, Liechtenstein.

The solutions are routinely applied after full dental restoration and after a prophylaxis of all tooth surfaces. The initial application, by either brush or cotton pellet, is the chlorhexidine solution, followed immediately by application of the sealant.

The initial chlorhexidine treatment may repeated on a predetermined dosing schedule. The preferred predetermined dosing schedule is weekly for one month (*i.e.*, for a total of four times) followed by additional treatment at six month intervals for as long as the clinician deems appropriate. Clinical studies have shown that, for xerostomic patients, every 6 months is sufficient to meaningfully reduce caries progression.

An adult dose of solution per treatment ranges from about 40 to 60 mg of chlorhexidine in the oral cavity. As noted above, it is preferred in the practice of this invention, that the concentration of chlorhexidine be 10% or less. This amount has been found to be effective for extended periods of time when applied in the preferred dosing schedule. Advantageously, less chlorhexidine reduces adverse side effects such as toxicity, staining, and loss of taste acuity.

The varnish and sealing solutions are stored under refrigerated conditions (preferably ranging from about 2 to 8 degrees Celsius) prior to application to avoid degradation, and can be used for a period of up to 30 months from the date of manufacture.

Although the invention has been described in terms of treatment of humans, particularly humans at risk for root caries and/or periodontal disease, it is to be specifically understood that the invention could find application in the veterinarian field. Therefore, the term "patients" includes mammals, such as cats, dogs, and horses.

### Detailed Description

### Clinical Results

Due to commercial interest in the use of chlorhexidine, multiple controlled studies were undertaken to determine the efficacy of the use of the antimicrobial drug, chlorhexidine, in controlling caries, and not just in controlling an *S. mutans* infection. The results of these studies, reported hereinbelow, were unexpected.

In a controlled clinical study reported hereinbelow involving older age groups, selected for participation due to high levels of *S. mutans,* a solution of chlorhexidine followed by a dental sealant layer showed, surprisingly, that these solutions could meaningfully prevent the incidence of cementum destruction, but produced no comparable results for enamel surfaces. Still, surprisingly, in the study group of older adults (see Clinical Study 1), this treatment controlled both destruction of the cementum and tissue inflammation at the gingival margin.

As indicated, these results were unexpected since the literature associated with the use of chlorhexidine solution and a sealant, including the patent literature as exemplified in U. S. Patent Nos. 4,496, 322 and 4,883,534, reported that these topical applications reduced the key microbial pathogen for caries, *S. mutans,* to low levels for long periods of time, and hence, it was believed that the enamel surfaces would be protected by this means. The literature has long reported a link between enamel caries and *S. mutans* infections.

Furthermore, the microorganism *Lactobacillus,* has been implicated in the dental literature as the key pathogen associated with the demineralization and destruction of cementum. However, it is known that *Lactobacillus* is resistant to the antimicrobial effects of chlorhexidine. Therefore, it was expected that the cementum would not be afforded meaningful protection from demineralization by the application of chlorhexidine. See, Cleghorn, et al., J. Dent. Res., Vol. 68, pages 1146-1150 (1989); Baker, et al., J. Dent. Res., Vol. 66, No. 6, pp. 1099-1106 (1987).

In addition to the foregoing, it is surprising that the supragingival application of chlorhexidine varnish prevents gingival inflammation since the known periodontopathic organisms responsible for the inflammation reside typically in the subgingival plaque.

In accordance with the principles of the present invention, and for the purposes of the clinical studies reported herein, a topical solution containing 10% (w/v) chlorhexidine, 20% (w/v) Sumatra benzoin, and 70% (w/v) ethanol was applied to the appropriate area of the tooth surface, as defined hereinabove, followed immediately by application of a sealant which was a solution containing 29% (w/v) medical-grade polyurethane in 49 % (w/v) acetone and 22% (w/v) ethyl acetate.

This treatment involves the sequential application of the chlorhexidine and then sealant solutions by brush or cotton pellet to the entire surface of the external crown and root of each tooth. In a practical embodiment of the invention, all teeth should be fully restored with no active caries lesions, then cleaned and dried before treatment. The application of the solutions to the soft tissues in the oral cavity should be avoided.

This treatment was repeated weekly for a period of a month. The patient was retreated in accordance with this method at six month intervals thereafter.

### Clinical Study 1

In one multi-center controlled study involving 236 adults (average age 58.7 years) with xerostomia ("dry mouth," measured by unstimulated salivary flow of less than 0.08 cumin) due to chronic systemic medication, the treatment in accordance with the principles of the invention, as set forth hereinabove, was shown to prevent cementum destruction at a statistically-significant level and to control gingival inflammation at a statistically significant level, different from the control groups. The results are shown in tabular form in Table 2.

Entry criteria for participation in this study included elevated levels of *S. mutans* (average titer of 11.5 x 10⁶ cfu per ml saliva), active decay and limited salivary flow (mean unstimulated salivary flow below 0.08 ml per minute) The participants were monitored over a one year period.

**TABLE 2**

| Reduction of Caries Increment on the Enamel and Cementum of Adult Patients over One Year in a Controlled Clinical Trial | | | |
|---|---|---|---|
| Group | Caries Increment | % Reduction active vs. placebo | p Value (based on a two-sided test, last observation carried forward, intent to treat analysis) |
| Active enamel | 1.79 | 14.4% | 0.0644 |
| Placebo enamel | 2.09 | | |
| Active cementum | 0.77 | 40.8% | 0.0249 |
| Placebo cementum | 1.30 | | |

As shown in Table 2, active root caries were reduced by 40% as compared to a placebo (p value of 0.0249). Periodontal health was assessed using conventional techniques. Significant bleeding scores (p = 0.015) and plaque accumulation (p =0.003) explains the observed reduction in gingival inflammation. The treatment did not reduce coronal caries at a threshold which is statistically significant.

### Other Clinical Studies

In another study, 1265 adolescents (between 11 and 13 in age at baseline) at risk for enamel caries were observed over a three year period. Entry criteria included elevated levels of *S. mutans* (average titer over 250,000 cfu/ml saliva) and a history of decay. No overall treatment effect was observed between four treatment groups (active, placebo, negative, and positive controls). Most notably, the treatment did not reduce *S. mutans* levels for time periods described in the scientific literature.

In still another study wherein 210 youngsters (ages 6-7 at baseline) and young adolescents (ages 10-11 at baseline), at risk for enamel caries, were observed over a three year period, similar results were obtained. There was no significant reduction in enamel caries.

## Claims

1. Use of the antimicrobial agent chloroxidine in the form of a nontoxic topical solution comprising up to and including 10% (w/v) of a chlorhexidine salt for the manufacture of a medicament for preventing root caries and/or for preventing and controlling gingival inflammation caused by root caries or by an infection with *Lactobacillus sp.* in a patient population of older adults or adults or others who have salivary hypofunction when the medicammt is used to contact the tooth surface from the cementum down the gingival margin, including any exposed dentine between the enamel and the cementum.

2. Use according to claim 1, wherein the chlorhexidine salt is selected from chlorhexidine acetate, chlorhexidine hydrochloride and chlorhexidine gluconate.

3. Use according to claim 1 or claim 2, wherein the topical solution is a film-forming solution comprising means of sustained release of chlorhexidine,

4. Use according to claim 3, wherein the topical solution comprise 10% (w/v) chlorhexidine, 20% (w/v) Sumatra benzoin and 70% (w/v) ethanol.

5. Use according to any one of claims 1 to 4, wherein said medicament comprising 40 to 60 mg chlorhexidine per dose.

6. Use of the microbial agent chlorhexidine in the form of a nontoxic topical film-forming solution comprising 10% (w/v) chlorhexidine, 20% (w/v) Sumatra benzoin, and 70% (w/v) ethanol for the manufacture of a medicament for preventing root caries and/or for preventing and controlling gingival inflammation caused by root caries or an infection with *Lactobacillus sp.* in a patient population of older adults or adult or others who have salivary hypofunction when the medicament is used to contact the tooth surface from the cementum down to the gingival margin, including any exposed dentine between the enamel and the cementum.

## Patentansprüche

1. Anwendung der antimikrobiellen Substanz Chlorhexidin in Form einer nichttoxischen topischen Lösung umfassend bis zu einschließlich 10% (w/v) Chlorhexidinsalz für die Herstellung eines Medikaments für die Vorbeugung vor Wurzelkaries und/oder die Vorbeugung und Eindämmung von Zahnfleischentzündung, die durch Wurzelkaries oder durch eine Infektion mit Lactobacillus sp. verursacht wird, bei einer Patientenpopulation bestehend aus älteren Erwachsenen oder Erwachsenen oder anderen, die eine Speicheldrüsenunterfunktion haben, wenn das Medikament verwendet wird, um die Zahnfläche vom Zement bis hinunter zum Zahnfleischrand, einschließlich jegliches freiliegendes Dentin zwischen dem Schmelz und dem Zement, zu berühren.

2. Anwendung nach Anspruch 1, wobei das Chlorhexidinsalz aus Chlorhexidinacetat, Chlorhexidinhydrochlorid und Chlorhexidinglukonat gewählt wird.

3. Anwendung nach Anspruch 1 oder 2, wobei die topische Lösung eine filmbildende Lösung ist, die Mittel aus mit konstanter Geschwindigkeit freigegebenem Chlorhexidin umfasst.

4. Anwendung nach Anspruch 3, wobei die topische Lösung 10% (w/v) Chlorhexidin, 20% (w/v) Sumatra-Benzoin und 70% (w/v) Ethanol umfasst.

5. Anwendung nach einem der vorangegangenen Ansprüche 1 bis 4, wobei das Medikament 40 bis 60 mg Chlorhexidin pro Dosis umfasst.

6. Anwendung der antimikrobiellen Substanz Chlorhexidin in Form einer nichttoxischen filmbildenden Lösung umfassend 10% (w/v) Chlorhexidin, 20% (w/v) Sumatra-Benzoin und 70% (w/v) Ethanol für die Herstellung eines Medikaments für die Vorbeugung vor Wurzelkaries und/oder die Vorbeugung und Eindämmung von Zahnfleischentzündung, die durch Wurzelkaries oder durch eine Infektion mit Lactobacillus sp. verursacht wird, bei einer Patientenpopulation bestehend aus älteren Erwachsenen oder Erwachsenen oder anderen, die eine Speicheldrüsenunterfunktion haben, wenn das Medikament verwendet wird, um die Zahnfläche vom Zement bis hinunter zum Zahnfleischrand, einschließlich jegliches freiliegendes Dentin zwischen dem Schmelz und dem Zement, zu berühren.

## Revendications

1. Utilisation de l'agent antimicrobien chlorhexidine sous la forme d'une solution topique non toxique comprenant jusqu'à 10 % (poids/volume) inclus d'un sel de chlorhexidine pour la fabrication d'un médicament destiné à prévenir les caries radiculaires et/ou pour prévenir et maîtriser l'inflammation gingivale due à des caries radicalaires ou par une infection par *Lactobacillus sp.,* chez une population de patients adultes âgés ou adultes ou autres ayant une hypofonction salivaire lorsque le médicament est utilisé pour être en contact avec la surface de la dent à partir du cément en descendant jusqu'au bord gingival, comprenant toute dentine exposée entre l'émail et le cément.

2. Utilisation selon la revendication 1, dans laquelle le sel de chlorhexidine est choisi parmi l'acétate de chlorhexidine, le chlorhydrate de chlorhexidine et le gluconate de chlorhexidine.

3. Utilisation selon la revendication 1 ou la revendication 2, dans laquelle la solution topique est une solution filmogène comprenant un moyen de libération prolongée de chlorhexidine.

4. Utilisation selon la revendication 3, dans laquelle la solution topique comprend 10 % (poids/volume) de chlorhexidine, 20 % (poids/volume) de benzol de Sumatra et 70 % (poids/volume) d'éthanol.

5. Utilisation selon l'une quelconque des revendications 1 à 4, dans laquelle ledit médicament comprenant 40 à 60 mg de chlorhexidine par dose.

6. Utilisation de l'agent antibactérien chlorhexidine sous la forme d'une solution filmogène topique non toxique comprenant. 10 % (poids/volume) de chlorhexidine, 20 % (poids/volume) de benzoïne de Sumatra et 70 % (poids/volume) d'éthanol dans la fabrication d'un médicament permettant de prévenir les caries radicalaires et/ou de prévenir et maîtriser l'inflammation gingivale due à des caries radicalaires ou par une infection par *Lactobacillus sp.*, chez une population de patients adultes âgés ou adultes ou autres ayant une hypofonction salivaire lorsque le médicament est utilisé pour être en contact avec la surface de la dent à partir du cément en descendant jusqu'au bord gingival, comprenant toute dentine exposée entre l'émail et le cément.
